(19) Europäisches Patentamt

European Patent Office

Office européen des brevets

(11) **EP 1 054 251 A1**

(12) **EUROPEAN PATENT APPLICATION**
published in accordance with Art. 158(3) EPC

(43) Date of publication:
22.11.2000 Bulletin 2000/47

(51) Int. Cl.[7]: **G01N 21/35**

(21) Application number: 98901092.1

(86) International application number:
**PCT/JP98/00427**

(22) Date of filing: 02.02.1998

(87) International publication number:
**WO 99/39187 (05.08.1999 Gazette 1999/31)**

(84) Designated Contracting States:
**DE FR GB**

(71) Applicant: **Hitachi, Ltd.**
**Chiyoda-ku, Tokyo 101-8010 (JP)**

(72) Inventors:
• **TAKEZAWA, Yoshitaka**
**Ibaraki 312-0001 (JP)**

• **ITOO, Yuuzoo**
**Ibaraki 310-0851 (JP)**
• **KATAGIRI, Junichi**
**Ibaraki 311-0106 (JP)**

(74) Representative:
**Beetz & Partner**
**Patentanwälte**
**Steinsdorfstrasse 10**
**80538 München (DE)**

(54) **METHOD AND DEVICE FOR OIL DETERIORATION DIAGNOSIS**

(57) An oil deterioration diagnosing method and an apparatus for carrying out the same uses an optical sensor capable of determining a degree of deterioration of oil on the basis of transmission losses of near-infrared rays of two different wavelengths and the transmission loss difference between the transmission losses. The degree of deterioration of the oil can be determined without being affected by variable measuring temperature and the original color of the oil.

F I G. 1

1···OIL    2···ENGINE    3···OIL LEVEL GAGE
4···OPTICAL FIBER CABLE    5···LIGHT SOURCE UNIT
6···LIGHT RECEIVING UNIT
7···ARITHMETIC AND CONTROL UNIT
8···METER PANEL    9···ALARM LAMP

## Description

TECHNICAL FIELD

The present invention relates to a method of diagnosing the deterioration of oil for lubricating automotive engines, compressors, gears and the like, and a diagnostic apparatus for carrying out the same.

BACKGROUND ART

A known method of diagnosing the deterioration of oil proposed in Japanese Patent Laid-open No. 3-111741 employs an optical sensor which determines the amount of carbon particles contained in the oil from the intensity of an evanescent wave varying according to the concentration of particles in the oil. Another known method of diagnosing the deterioration of oil proposed in Japanese Patent Laid-open No. 8-62207 employs a technique which uses two kinds of radiation of different wavelengths, i.e., visible radiation and near-infrared radiation, and determines the deterioration of the oil from the absorbance of the oil.

However, the output of the optical sensor varies in a wide range according to the variation of the temperature of the engine oil varying in a wide range according to the operating condition of the engine. The diagnostic performance of the method using visible radiation and near-infrared radiation is subject to the original color of the oil dependent on additives contained in the oil, and the method is incapable of accurate diagnosis. It is an object of the present invention to solve the foregoing problems and to provide an optical method of diagnosing the deterioration of oil and a diagnostic apparatus for carrying out the same, not subject to the influence of temperature variation and the original color of the oil.

DISCLOSURE OF INVENTION

The inventors of the present invention examined the relation between the degree of deterioration of oil, such as an automotive engine oil, and the light transmission loss spectral characteristic per unit length of near-infrared radiation, and found that there is a correlation between the slope of a light transmission loss spectrum of short-wavelength near-infrared radiation and the level of the base line of the light transmission loss spectrum of long-wavelength near-infrared radiation, and the amount of sludge (amount of insoluble components), dynamic viscosity and total acid number. The present invention has been made on the basis of such a finding. The gist of the present invention is as follows.

(1) A method of diagnosing the deterioration of oil and a diagnostic apparatus for carrying out the same guides at least two kinds of light rays of different wavelengths emitted by two different monochromatic light sources into oil by an illuminating light guiding member, guides the light rays guided by the illuminating light guiding member so as to travel a transmission distance a through the oil, guides the transmitted light rays traveled through the oil by a received light guiding member, disposed opposite to the illuminating light guiding member, to a light receiving unit, calculates light transmission losses per unit length ($\alpha \cdot$ dB/mm) of the two kinds of light rays and the light transmission loss difference ($\Delta\alpha \cdot$ dB/mm) between the light transmission losses per unit length of the two kinds of light rays by an arithmetic and control unit, and determines the degree of deterioration of the oil through the comparison of the light transmission losses and the light transmission loss difference with previously stored data (master curves) representing the relation between the degree of deterioration of the oil and light transmission losses and the relation between the degree of deterioration of the oil and the light transmission loss difference by the arithmetic and control unit.

Laser diodes (LDs) or light-emitting diodes (LEDs) which emit light rays respectively having peak wavelengths in the range of 800 nm to 1500 nm are readily available, have long life and stable ability, and are suitable monochromatic light sources. LDs and LEDs that emit light rays of 800, 820, 830, 850, 940, 950, 1300, 1310 and 1550 nm in wavelength are particularly preferable. Overrange occurs sometimes in a photodetector included in a light receiving unit while the degree of deterioration is relatively low when a light source that emits light rays of a wavelength outside the foregoing wavelength range is used, which makes the measurement of the light rays impossible.

If the illuminating light guiding member is incorporated into an oil level gage for measuring the oil level of the automotive engine oil, any particular modification of the existing engine system is not necessary. Diagnostic result may be indicated as an alarm, i.e., one of self-checking functions, on the meter panel of the automobile or may be indicated on an indication unit attached to the grip of the oil level gage to enable the driver to recognize the condition of the engine oil when the driver executes a daily inspection routine.

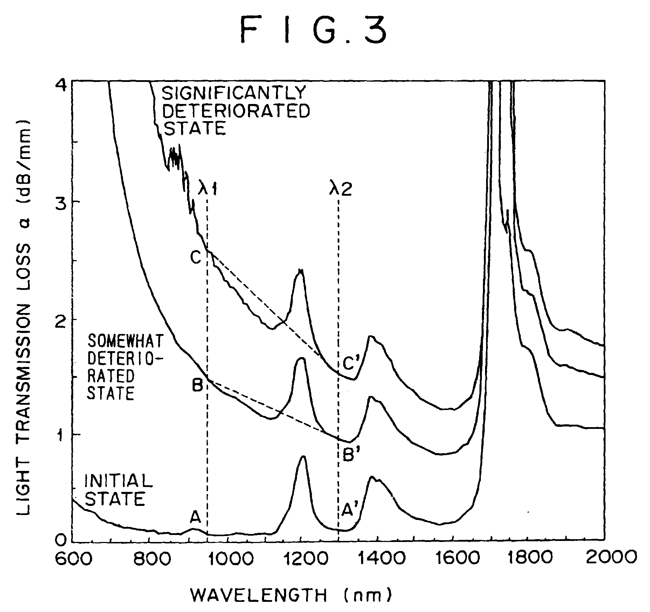

Generally, the degree of deterioration of the engine oil of an automobile and light transmission loss spectrum indicating light transmission losses per unit length are indicated by curves shown in Fig. 3.

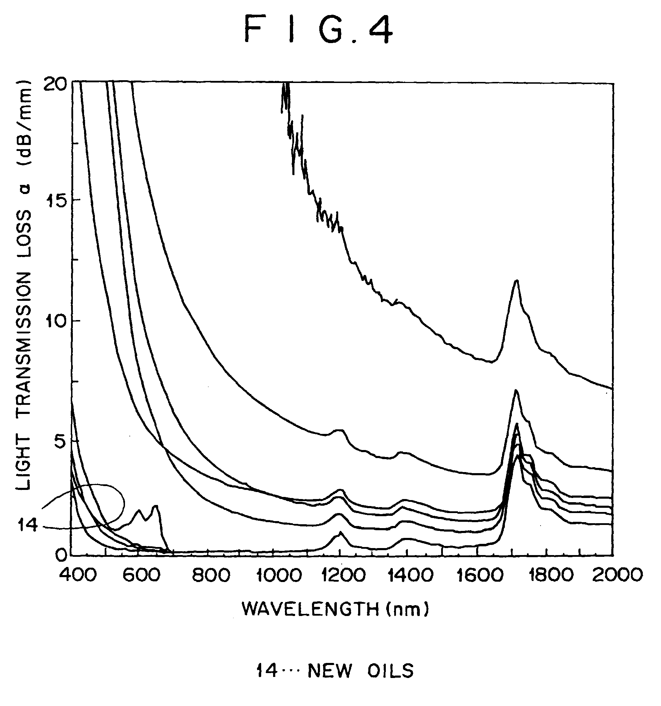

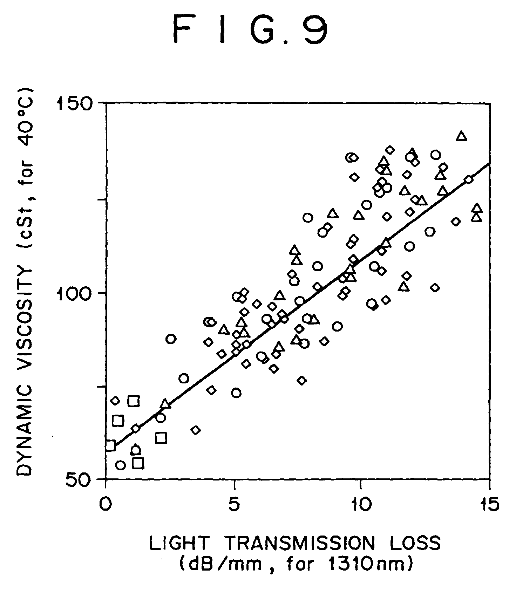

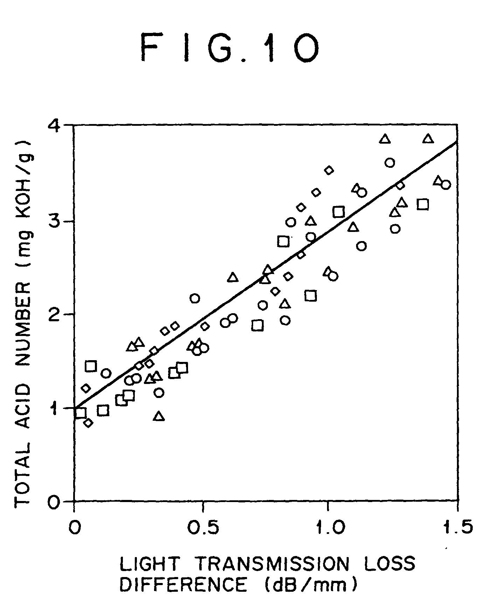

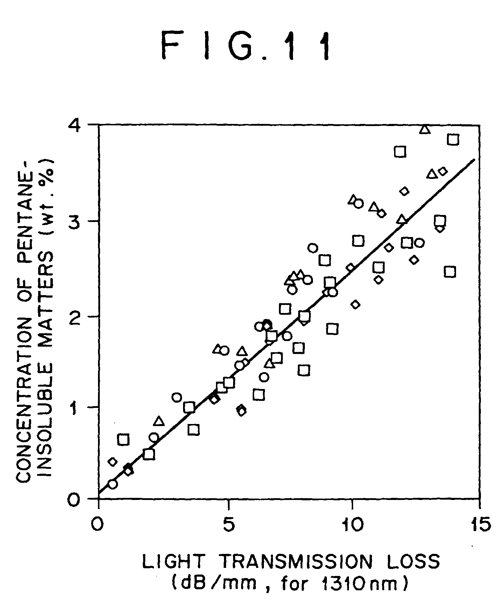

Since these light transmission loss spectrum are not affected by measuring temperature, the light transmission loss may be measured in carrying out a start-up inspection routine before using the automobile or may be measured while the automobile is in operation. As shown in Fig. 3, the light transmission losses of visible light rays in the visible region increase sharply and the darkness of the engine oil increases with the progress of deterioration. Therefore, overrange occurs while the degree of deterioration is relatively low. Thus, it was concluded that the visible light rays are unsuitable for the diagnosis of the deterioration of the oil. The increase in the spectrum from the side of short wavelength is caused principally by the increase of electronic transition absorption loss due to deterioration caused by thermal oxidation. The light transmission loss difference between two wavelengths indicates the inclination of a line A-A' in an initial stage of deterioration, the inclination of a line B-B when the oil is deteriorated in a middle degree of deterioration and the inclination of a line C-C' when the oil is deteriorated in a high degree of deterioration. Thus, the inclination increases with the progress of deterioration. As regards the light transmission loss of a base value, since the values of peaks near the points A', B' and C', i.e., harmonics absorption peaks of C-H bonds, do not change greatly, it is considered that light scattering loss due to the influence of sludge and the like (loss due to what is called Mie scattering) increases and the amount of insoluble matters can be measured. Fig. 4 shows light transmission loss spectrum of used engine oils used in different modes of use and four kinds of new engine oils 14. The four kinds of new oils contain different additives and hence have different colors, respectively. However, the values of the light transmission loss spectrum for wavelengths above 700 nm coincide perfectly, which signifies that the diagnosis of the condition of the oil can be achieved without being affected by the type of the oil if near-infrared radiation is used. Fig. 9 shows the relation between light transmission loss caused by engine oils used on practical automobiles differing from each other in distance traveled, type and mode of use with light rays of 1310 nm in wavelength, and dynamic viscosity at 40 °C by way of example. Fig. 10 shows the relation between the light transmission loss difference between light rays of 950 nm and 1310 nm in wavelength, and total acid number. Fig. 11 shows the relation between light transmission loss with light rays of 1310 nm and the concentration of pentane-insoluble matters. It is known from Figs. 9, 10 and 11 that each parameter correlates with the light transmission loss and the light transmission loss difference to a high degree.

Since there is a correlation between light transmission loss and light transmission loss difference varying with the progress of deterioration, and the parameters serving as measures of degree of deterioration of the oils, the deterioration of the physical properties of the oil can be diagnosed only by measuring light transmission loss and light transmission loss difference. As mentioned in Japanese Patent Laid-open No. 3-226651, it is usual to represent the degree of deterioration by reduced time $\theta$. It is considered that materials of different kinds of deterioration history having the same reduced time $\theta$ have the same degree of deterioration. Reduced time $\theta$ is defined by:

$$\theta = t \times \exp(-\Delta E/RT) \qquad (1)$$

where $\Delta E$ (J/mol) is apparent activation energy of deterioration, R (J/K/mol) is gas constant, T (K) is absolute temperature of deterioration, and t (h) is time of deterioration. The value of $\Delta E$ of the deterioration of the oil can easily be calculated by using the Arrhenius equation. Suppose that life equivalent reduced time is $\theta_0$ at a predetermined life end point of the oil. Then, the difference $\Delta\theta$ between the life equivalent reduced time $\theta_0$ and an reduced time $\theta$ determined on the basis of measurements is an equivalent time corresponding to remaining life, which can be used as a measure of deterioration. The remaining life $\Delta\theta$ (h) is expressed by:

$$\Delta\theta = \theta_0 - \theta \qquad (2)$$

If average operating temperature of the oil after the time t is determined by using Expression (2), time $\Delta t$ ($t_0$ - t) corresponding to remaining life can be determined.

BRIEF DESCRIPTION OF DRAWINGS

Fig. 1 is a typical view of an engine oil deterioration diagnosing apparatus for diagnosing the deterioration of an engine oil used in an automobile;
Fig. 2 is a side elevation of an optical sensing device incorporated into an oil gage;
Fig. 3 is a graph of assistance in explaining the variation of a light transmission loss spectrum with the deterioration of the engine oil;
Fig. 4 is a graph showing light transmission loss spectrum obtained by using engine oils used in engines operated in different modes of operation and new engine oils;
Fig. 5 is a graph of an example of a diagnostic master curve using light transmission loss difference as a parameter;
Fig. 6 is a graph of an example of a diagnostic master curve using light transmission loss as a parameter;
Fig. 7 is a flow chart of an oil deterioration diagnosing routine;
Fig. 8 is a side elevation of an optical sensing device incorporated into an oil gage;
Fig. 9 is a graph showing the relation between light transmission losses caused by engine oils used in engines operated in different modes of operation, and the dynamic viscosities of the engine oils;
Fig. 10 is a graph showing the relation between light transmission losses caused by engine oils used in engines operated in different modes of operation, and the total acid numbers of the engine oils;
Fig. 11 is a graph showing the relation between light transmission losses caused by engine oils used in engines operated in different modes of operation, and the concentrations of pentane-insoluble matters; and

Fig. 12 is a side elevation of a sensor provided with an indication unit attached to the grip of an oil level gage.

BEST MODE FOR CARRYING OUT THE INVENTION

Preferred embodiments of the present invention will be described hereinafter with reference to the accompanying drawings. It is to be noted the present invention is not limited in its practical application to the preferred embodiments specifically described herein.

First Embodiment

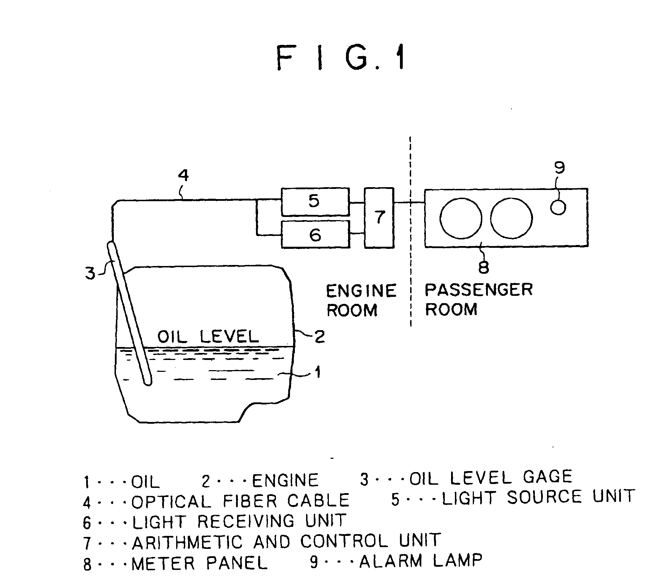

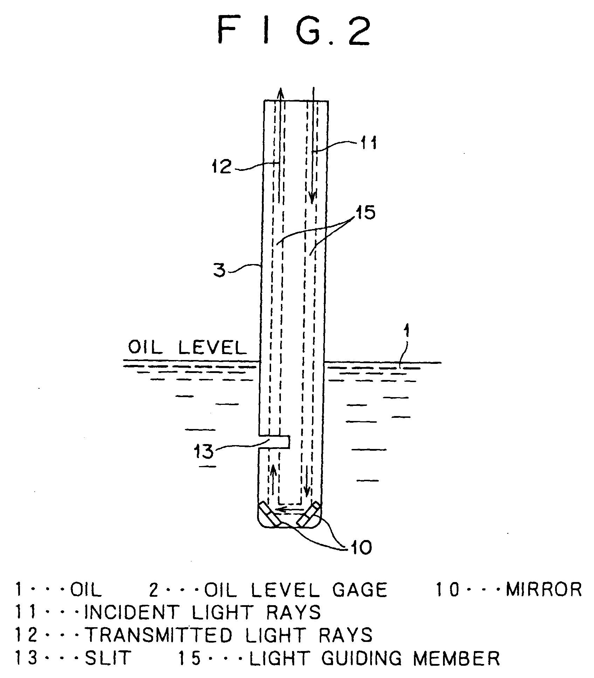

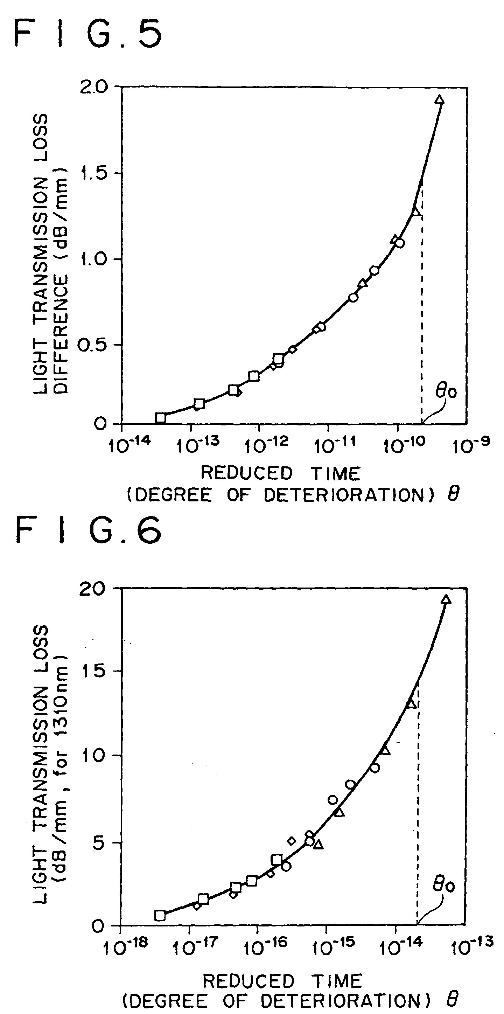

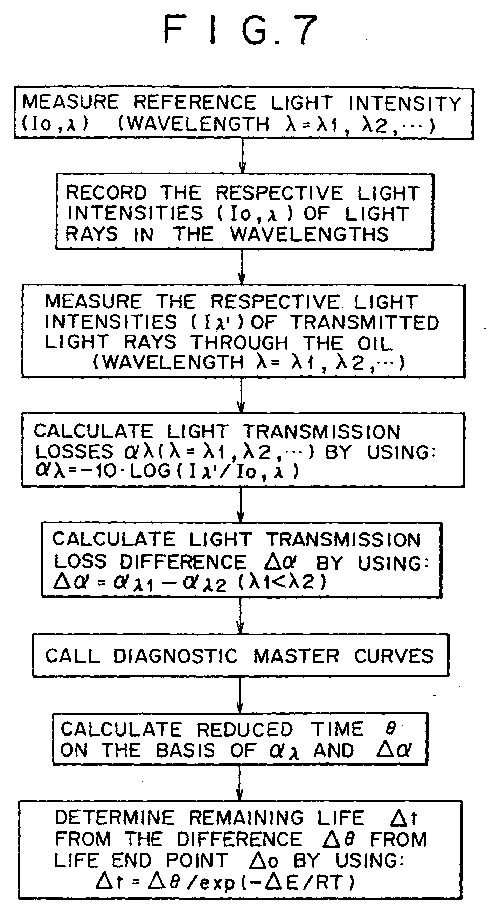

Fig. 1 is a typical view of an engine oil deterioration diagnosing apparatus for diagnosing the deterioration of an engine oil used in an automobile, and Fig. 7 is a flow chart of an oil deterioration diagnosing routine. Referring to Fig. 1, an arithmetic and control unit 7 comprises microprocessor comprising a measured data memory, and a read-only memory. The arithmetic and control unit 7 changes the wavelength of light rays emitted by a light source unit, measures the intensity of received light, and carries out arithmetic operations. The embodiment will be described on an assumption that light rays of two different wavelengths are used. The light source unit has a light-emitting diode (LED) which emits light rays of a wavelength $\lambda 1$ of 950 nm and a laser diode (LD) which emits light rays of a wavelength $\lambda 2$ of 1310 nm. Reference light intensity ($I_0, \lambda$) of each of the light rays of different wavelengths is measured. Incident light rays 11 of the wavelength $\lambda 1$ travel through an optical fiber cable 4 to an oil level gage 3. Fig. 2 shows the internal construction of the oil level gage 3. The incident light rays 11 are transmitted by a light guiding member 15 arranged in the oil level gage 3, are deflected by mirrors 10, travel across a slit 13 of an optical path length of 1.0 mm. The optical path length of the slit 13 may be a length in the range of 0.5 to 2.0 mm. The incident light rays 11 travel through an oil 1 filling the slit, and transmitted light rays 11 transmitted through the slit 13 travel through a light guiding member 15 in transmitted light rays 12 to a light receiving unit 6. The intensity of the transmitted light rays of the wavelength $\lambda 1$ is measured by the light receiving unit 6, and the arithmetic and control unit 7 calculates a light transmission loss and stores the calculated light transmission loss. Similarly, incident light rays 11 of the wavelength $\lambda 2$ travel through the slit 13 and travel in transmitted light rays to the light receiving unit 6. The intensity of the transmitted light rays 11 of the wavelength $\lambda 2$ is measured and the arithmetic and control unit 7 calculates a light transmission loss of the light rays of the wavelength $\lambda 2$ and stores the calculated light transmission loss of the light rays of the wavelength $\lambda 2$. The arithmetic and control unit 7 calculates an equivalent time corresponding to the degree of deterioration of the oil by using previously stored master curves as shown in Figs. 5 and 6 representing the relation between degree of deterioration of oil and the light transmission loss and the relation between degree of deterioration of oil and light transmission loss difference, and indicates the result of calculation by an alarm lamp installed in the automobile. This inspection is executed by a self-checking system after the start of the engine.

Second Embodiment

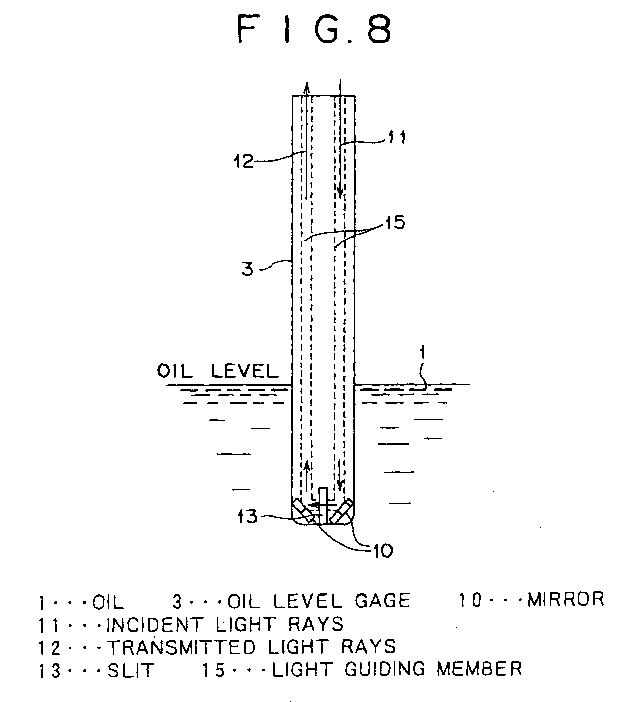

A second embodiment, similarly to the first embodiment, uses an oil level gage 3 having an internal construction as shown in Fig. 8. The second embodiment is provided with a light-emitting diode (LED) as a light source which emits light rays of a wavelength $\lambda 1$ of 940 nm, and a laser diode (LD) as a light source which emits light rays of a wavelength $\lambda 2$ of 1550 nm. The reference light intensity ($I_0, \lambda$) of the light rays 11 of each wavelength is measured. The light rays 11 of the wavelength $\lambda 1$ travel through an optical fiber cable 4 to an oil level gage 3. The oil level gage 3 has an internal construction as shown in Fig. 2. The incident light rays 11 travel through a light guiding member 15, are deflected by a mirror 10, travel through the oil 1 filling a slit 13 of 0.5 mm in optical path length, and travel in transmitted light rays 12 through the light guiding member 15 to a light receiving unit 6. The light receiving unit 6 measures the intensity of the transmitted light rays of the wavelength $\lambda 1$, and an arithmetic and control unit 7 calculates a light transmission loss and stores the calculated light transmission loss of the light rays of the wavelength $\lambda 1$. Similarly, the intensity of the transmitted light rays 11 of the wavelength $\lambda 2$ is measured and the arithmetic and control unit 7 calculates a light transmission loss of the light rays of the wavelength $\lambda 2$ and stores the calculated light transmission loss. The arithmetic and control unit 7 calculates an equivalent time corresponding to the degree of deterioration of the oil by using previously stored master curves as shown in Figs. 5 and 6 representing the relation between degree of deterioration of oil and light transmission loss and the relation between degree of deterioration of oil and light transmission loss difference, and indicates the result of calculation by an alarm lamp installed in the automobile. This inspection is executed by a self-checking system after the start of the engine.

Third Embodiment

A third embodiment, similarly to the first embodiment, employs an oil level gage 3 of an internal construction as shown in Fig. 8. The third embodiment is provided with a light-emitting diode (LED) as a light source which emits light rays of a wavelength $\lambda 1$ of 850 nm, and a laser diode (LD) as a light source which emits light rays of a wavelength $\lambda 2$ of 1550 nm. The reference light intensity ($I_0, \lambda$) of the light rays of each wavelength is measured. The light rays 11 of the wavelength $\lambda 1$

travel through an optical fiber cable 4 to the oil level gage 3. The oil level gage 3 has an internal construction as shown in Fig. 2. The incident light rays 11 travel through a light guiding member 15, are deflected by mirrors 10, travel through an oil 1 filling a slit 13 of 1.5 mm in optical path length, and travel in transmitted light rays 12 through the light guiding member 15 to a light receiving unit 6. The light receiving unit 6 measures the intensity of the transmitted light rays of the wavelength $\lambda 1$, and an arithmetic and control unit 7 calculates a light transmission loss and stores the calculated light transmission loss of the light rays of the wavelength $\lambda 1$. Similarly, the intensity of the transmitted light rays 11 of the wavelength $\lambda 2$ is measured and the arithmetic and control unit 7 calculates a light transmission loss of the light rays of the wavelength $\lambda 2$ and stores the calculated light transmission loss. The arithmetic and control unit 7 calculates an equivalent time corresponding to the degree of deterioration of the oil by using previously stored master curves as shown in Figs. 5 and 6 representing the relation between degree of deterioration of oil and light transmission loss and the relation between degree of deterioration of oil and light transmission loss difference, and indicates the result of calculation by an alarm lamp installed in the automobile. This inspection is executed by a self-checking system after the start of the engine.

Fourth Embodiment

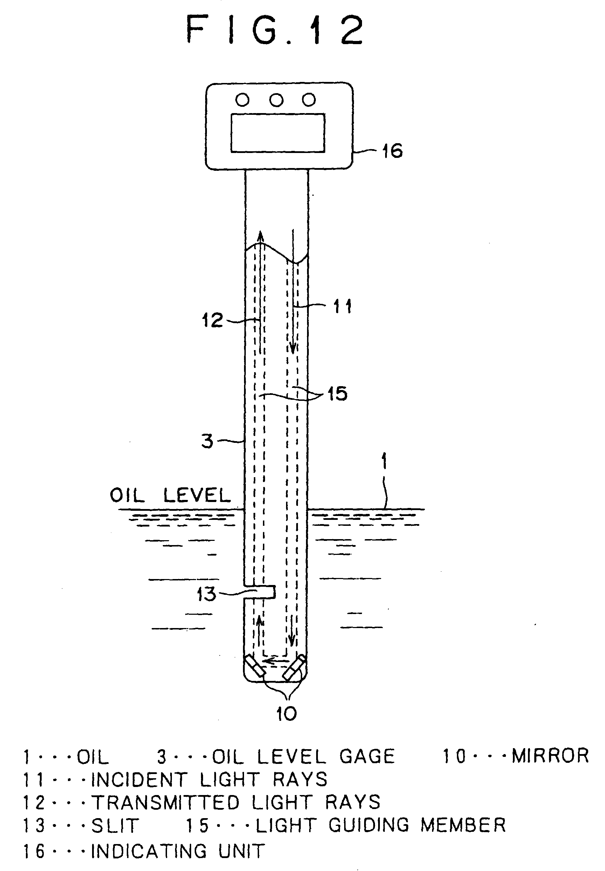

An engine oil deterioration diagnosing apparatus in a fourth embodiment according to the present invention is similar to that in the first embodiment. The engine oil deterioration diagnosing apparatus indicates the result of diagnosis on an indication unit attached to the grip of an oil level gage as shown in Fig. 12. This inspection is executed as a part of daily inspection routine to be carried out before using the automobile.

INDUSTRIAL APPLICABILITY

According to the present invention, the degree of deterioration of oil used for lubricating the engine of an automobile, compressor or gears can be diagnosed without being affected by measuring temperature and the original color of the oil. The engine oil deterioration diagnosing apparatus can be formed in either an on-vehicle type or a portable type.

**Claims**

1.  An oil deterioration diagnosing method comprising the steps of:

    guiding at least two kinds of light rays of different wavelengths emitted by two different monochromatic light sources into oil by an illuminating light guiding member;

    guiding the light rays guided by the illuminating light guiding member so as to travel a transmission distance a through the oil;
    guiding the transmitted light rays traveled through the oil by a receiving light guiding member disposed opposite to the illuminating light guiding member to a light receiving unit;
    calculating light transmission losses per unit length ($\alpha \cdot$ dB/mm) of the two kinds of light rays and the light transmission loss difference ($\Delta\alpha \cdot$ dB/mm) between the light transmission losses per unit length of the two kinds of light rays by an arithmetic and control unit; and
    determining the degree of deterioration of the oil through the comparison of the light transmission losses and the light transmission loss difference with previously stored data (master curves) representing the relation between the degree of deterioration of the oil and light transmission losses and light transmission loss difference by the arithmetic and control unit.

2.  The oil deterioration diagnosing method according to claim 1, wherein the monochromatic light sources are laser diodes or light-emitting diodes which emit light rays respectively having peak wavelengths in the range of 800 nm to 1500 nm.

3.  The oil deterioration diagnosing method according to claim 1, wherein the illuminating light guiding member is incorporated into an oil level gage of the engine of an automobile.

4.  An oil deterioration diagnosing apparatus comprising:

    a light source unit comprising at least two monochromatic light sources capable of emitting light rays respectively having different wavelengths;
    an illuminating light guiding member for guiding the light rays emitted by the light source unit into oil;
    a receiving light guiding member disposed opposite to the illuminating light guiding member to guide the transmitted light rays to the outside after the light rays travel a transmission distance a through the oil;
    a light receiving unit for measuring the respective intensities of the transmitted light rays by the receiving light guiding member; and
    an arithmetic and control unit which calculates light transmission losses per unit length ($\alpha \cdot$ dB/mm) of the two kinds of light rays and the light transmission loss difference ($\Delta\alpha \cdot$ dB/mm) between the light transmission losses per unit length of the two kinds of light rays on the basis of the measured intensities of the transmitted light rays, and determining the degree of deterioration of the oil through the comparison of the light transmission losses and the light transmission loss difference with previously stored data (master curves) representing the relation between the degree of deterioration of the oil and light transmission losses and the relation between the degree of deterioration of the oil and light transmission loss difference.

5. The oil deterioration diagnosing apparatus according to claim 4, wherein the monochromatic light sources are laser diodes or light-emitting diodes which emit light rays respectively having peak wavelengths in the range of 800 nm to 1500 nm.

6. The oil deterioration diagnosing apparatus according to claim 4, wherein the illuminating light guiding member is incorporated into an oil level gage included in an engine included in an automobile.

7. The oil deterioration diagnosing apparatus according to claim 4, wherein a degree of deterioration of the oil determined by the arithmetic and control unit is indicated by an indication unit attached to the grip of an oil level gage included in an engine included in an automobile.

8. The oil deterioration diagnosing apparatus according to claim 4, wherein a degree of deterioration of the oil determined by the arithmetic and control unit is indicated by an indication unit installed on a meter panel placed in an automobile.

# F I G. 1

1···OIL    2···ENGINE    3···OIL LEVEL GAGE
4···OPTICAL FIBER CABLE    5···LIGHT SOURCE UNIT
6···LIGHT RECEIVING UNIT
7···ARITHMETIC AND CONTROL UNIT
8···METER PANEL    9···ALARM LAMP

# F I G. 2

OIL LEVEL

1···OIL     2···OIL LEVEL GAGE     10···MIRROR
11···INCIDENT LIGHT RAYS
12···TRANSMITTED LIGHT RAYS
13···SLIT     15···LIGHT GUIDING MEMBER

# F I G . 3

# F I G . 4

14 ··· NEW OILS

# F I G . 5

# F I G . 6

# F I G. 7

MEASURE REFERENCE LIGHT INTENSITY
$(I_0, \lambda)$ (WAVELENGTH $\lambda = \lambda 1, \lambda 2, \cdots$)

RECORD THE RESPECTIVE LIGHT
INTENSITIES $(I_0, \lambda)$ OF LIGHT
RAYS IN THE WAVELENGTHS

MEASURE THE RESPECTIVE LIGHT
INTENSITIES $(I_{\lambda}')$ OF TRANSMITTED
LIGHT RAYS THROUGH THE OIL
(WAVELENGTH $\lambda = \lambda 1, \lambda 2, \cdots$)

CALCULATE LIGHT TRANSMISSION
LOSSES $\alpha \lambda (\lambda = \lambda 1, \lambda 2, \cdots)$ BY USING:
$\alpha \lambda = -10 \cdot LOG (I_{\lambda}' / I_0, \lambda)$

CALCULATE LIGHT TRANSMISSION
LOSS DIFFERENCE $\Delta \alpha$ BY USING:
$\Delta \alpha = \alpha_{\lambda 1} - \alpha_{\lambda 2}$ $(\lambda 1 < \lambda 2)$

CALL DIAGNOSTIC MASTER CURVES

CALCULATE REDUCED TIME $\theta$
ON THE BASIS OF $\alpha_{\lambda}$ AND $\Delta \alpha$

DETERMINE REMAINING LIFE $\Delta t$
FROM THE DIFFERENCE $\Delta \theta$ FROM
LIFE END POINT $\Delta_0$ BY USING:
$\Delta t = \Delta \theta / exp (-\Delta E / RT)$

# F I G. 8

1 ··· OIL    3 ··· OIL LEVEL GAGE    10 ··· MIRROR
11 ··· INCIDENT LIGHT RAYS
12 ··· TRANSMITTED LIGHT RAYS
13 ··· SLIT    15 ··· LIGHT GUIDING MEMBER

# F I G. 9

# F I G. 1 0

# F I G . 1 1

# F I G . 1 2

1 · · ·OIL     3 · · ·OIL LEVEL GAGE     10 · · ·MIRROR
11 · · ·INCIDENT LIGHT RAYS
12 · · ·TRANSMITTED LIGHT RAYS
13 · · ·SLIT     15 · · ·LIGHT GUIDING MEMBER
16 · · ·INDICATING UNIT

17

# INTERNATIONAL SEARCH REPORT

| International application No. |
|---|
| PCT/JP98/00427 |

**A. CLASSIFICATION OF SUBJECT MATTER**
Int.Cl⁶ G01N21/35

According to International Patent Classification (IPC) or to both national classification and IPC

**B. FIELDS SEARCHED**

Minimum documentation searched (classification system followed by classification symbols)
Int.Cl⁶ G01N21/00-21/01, G01N21/17-21/61

Documentation searched other than minimum documentation to the extent that such documents are included in the fields searched
Jitsuyo Shinan Koho          1922-1996   Toroku Jitsuyo Shinan Koho   1994-1998
Kokai Jitsuyo Shinan Koho   1971-1998   Jitsuyo Shinan Toroku Koho   1996-1998

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| Y | JP, 2-236440, A (Komatsu Ltd.), September 19, 1990 (19. 09. 90), Full text ; Figs. 1 to 7 (Family: none) | 1, 4 |
| Y | Microfilm of the specification and drawings annexed to the request of Japanese Utility Model Application No. 69209/1986 (Laid-open No. 180753/1987) (Nippon Radiator K.K.), November 17, 1987 (17. 11. 87), Full text ; Figs. 1 to 11 (Family: none) | 1-6 |
| Y | JP, 7-140074, A (Hitachi, Ltd.), June 2, 1995 (02. 06. 95), Full text ; Figs. 4, 6, 7 (Family: none) | 1-2, 4-5 |
| Y | JP, 2-49644, B2 (Tokyo Tatsuno Co., Ltd.), October 30, 1990 (30. 10. 90), Full text ; Figs. 1, 2 (Family: none) | 3, 6-7 |

[X] Further documents are listed in the continuation of Box C.   [ ] See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier document but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | | |
| "O" | document referring to an oral disclosure, use, exhibition or other means | "Y" | document of particular relevance; the claimed invention cannot be considered to involve an inventive step when the document is combined with one or more other such documents, such combination being obvious to a person skilled in the art |
| "P" | document published prior to the international filing date but later than the priority date claimed | "&" | document member of the same patent family |

| Date of the actual completion of the international search | Date of mailing of the international search report |
|---|---|
| April 28, 1998 (28. 04. 98) | May 12, 1998 (12. 05. 98) |

| Name and mailing address of the ISA/ Japanese Patent Office | Authorized officer |
|---|---|
| Facsimile No. | Telephone No. |

Form PCT/ISA/210 (second sheet) (July 1992)

# INTERNATIONAL SEARCH REPORT

| | International application No. |
|---|---|
| | PCT/JP98/00427 |

C (Continuation). DOCUMENTS CONSIDERED TO BE RELEVANT

| Category* | Citation of document, with indication, where appropriate, of the relevant passages | Relevant to claim No. |
|---|---|---|
| Y | JP, 7-12723, A (Mitsubishi Heavy Industries, Ltd.), January 17, 1995 (17. 01. 95), Page 1, left column, lines 2 to 24 ; page 7, right column, lines 35 to 40, Fig. 25 (Family: none) | 8 |

Form PCT/ISA/210 (continuation of second sheet) (July 1992)